# DEMANDE DE BREVET EUROPEEN

(11) **EP 2 556 854 A1**
(43) Date de publication de la demande: **13.02.2013**
(21) Numéro de dépôt: 12179690.8
(22) Date de dépôt: 08.08.2012
(51) Int. Cl.: A61M 39/28, F16K 7/06, A61M 5/168

(54) **Ligne de perfusion multivoie**

(30) Priorité: 09.08.2011 FR 1157255
(71) Demandeur: ACE Development Solution, 69380 Lissieu (FR)
(72) Inventeur: Delorme, Patrick, 69630 Chaponost (FR); Lopez, Georges-Antoine, 69130 Ecully (FR)
(74) Mandataire: Delorme, Nicolas

(57) **Abrégé**

Ligne de perfusion multivoie (1) comprenant
- plusieurs compartiments (3) susceptibles de maintenir chacun une tubulure (5) engagée dans une pince (8) d'obturation, chacun des compartiments (3) comportant un moyen de réception de la pince (8) conçu pour se déplacer entre:
une position d'obturation de la tubulure (5) dans laquelle la pince (8) insérée dans le moyen de réception compresse la tubulure (5), et
une position d'ouverture de la tubulure (5) dans laquelle la pince (8) libère la tubulure (5),

- plusieurs poches (4) destinées à contenir un fluide, chacune des poches (4) étant reliée à un compartiment (3) par l'intermédiaire d'une tubulure (5), chacune des tubulures (5) étant reliées en aval du boitier (2) à une tubulure principale (6) engagée dans la pompe (7), la pompe (7) régulant le débit du fluide dans la ligne (1) de perfusion, et
- des moyens de contrôle programmables de la position de chacune des pinces (8) d'obturation permettant le séquençage automatique de la perfusion du fluide des différentes poches (4) de la ligne (1).

## Description

La présente invention a pour objet une ligne de perfusion multivoie.

Certains traitements médicaux nécessitent l'administration de plusieurs substances médicales sous perfusion. Lorsque ces substances ne sont pas miscibles ou doivent être administrées de façon séquencée, un dispositif de perfusion multivoie est souvent utilisé. Toutefois, arrivé en fin d'injection d'une substance active, ces dispositifs nécessitent l'intervention d'un opérateur qualifié pour initier la séquence d'injection suivante, ce qui complique le déroulement de la perfusion et augmente le risque d'erreur de manipulation. De tels dispositifs présentent également le risque que le personnel soignant soit en contact avec les produits utilisés souvent très toxiques.

Un dispositif automatique simple à utiliser, qui permette une administration séquencée, sans intervention de personnel soignant et qui soit parfaitement étanche pour éviter tout risque de contact avec les substances toxiques est donc souhaitable.

A cet effet, la présente invention propose une ligne de perfusion multivoie caractérisé en ce qu'elle comprend :
- un boitier comprenant plusieurs compartiments susceptibles de maintenir chacun une tubulure pour fluide pourvue d'une pince d'obturation, chacun des compartiments comprenant un moyen de réception de la pince conçu pour se déplacer entre :
   une position d'obturation de la tubulure dans laquelle la pince insérée dans le moyen de réception compresse la tubulure de sorte à interrompre la circulation du fluide dans la tubulure et
   une position d'ouverture de la tubulure dans laquelle la pince libère la tubulure de sorte à autoriser la circulation du fluide dans la tubulure,
   le boitier comprenant des moyens de commande du déplacement de chaque moyen de réception,
- plusieurs poches destinées à contenir un fluide, chacune des poches étant reliée à un compartiment du boitier par l'intermédiaire d'une tubulure, chacune des tubulures étant reliées en aval du boitier à une tubulure principale, la pompe régulant le débit du fluide dans la ligne de perfusion, et
- des moyens de contrôle programmables de la position de chacune des pinces d'obturation permettant le séquençage automatique de la perfusion du fluide des différentes poches de la ligne de perfusion multivoie.

Ainsi, la présente invention propose une ligne de perfusion peu coûteuse et simple d'utilisation. Le séquençage automatique est obtenu par l'action des moyens de contrôle programmables et la pompe. En effet, la pompe réalise l'aspiration à partir de la tubulure principale quelle que soit la poche de fluide à perfuser, la sélection de la poche du fluide à perfuser étant obtenue par les moyens de contrôles programmés. Il est ainsi possible d'effectuer une injection de plusieurs fluides de différentes poches en même temps ou de manière décalée.

Par ailleurs, les moyens de commande du déplacement de chaque moyen de réception du boitier permettent ainsi le déplacement de la pince pour l'obturation et l'ouverture de la tubulure indépendamment pour chacun des compartiments du boitier ce qui sécurise l'administration séquencée et automatique du fluide de chacun des compartiments en fonction de la programmation effectuée des moyens de contrôle.

Les termes « pince d'obturation » correspondent au terme « clamp » souvent utilisé suivant la terminologie anglo-saxonne.

Selon une possibilité, les moyens de contrôle programmables sont intégrés à la pompe.

Avantageusement, la pompe est péristaltique.

De préférence, chacun des compartiments du boitier comprend une porte équipée de moyens de verrouillage aptes à verrouiller la porte pour interdire l'accès à la pince d'obturation en position d'ouverture de la tubulure. De la sorte, les erreurs de manipulation sont supprimées et l'administration du fluide est sécurisée. De même, les risques de contact des opérateurs avec les substances médicales contenues dans les fluides sont évités.

Selon une possibilité, le moyen de réception de la pince d'obturation comprend un tiroir mobile dans lequel la pince est insérée de sorte à faciliter le déplacement de la pince entre ses deux positions d'obturation et d'ouverture de la tubulure et chaque compartiment comprend des portées sur lesquelles le tiroir mobile peut se déplacer entre la position d'ouverture et la position d'obturation de la pince. Typiquement, la pince est dotée de dents qui viennent se clipser dans des orifices prévus dans le tiroir de sorte à assurer la solidarité de la pince et du tiroir au cours des différents déplacements. Par ailleurs, les portées ont pour effet de guider le déplacement du tiroir entre les deux positions.

Selon une variante, chaque compartiment comprend des moyens de rappel élastiques qui sollicitent le tiroir vers la position d'obturation de la pince. La tubulure est ainsi obturée même lorsque le tiroir est au repos, ce qui évite la délivrance accidentelle de fluide.

Les moyens de rappels élastiques sont typiquement constitués de ressorts.

Avantageusement, chaque compartiment comprend un moyen de commande du tiroir mobile entre une position inactive dans laquelle le tiroir est maintenu en position d'obturation de la pince par les moyens de rappel élastiques et une seconde position active dans laquelle le moyen de commande pousse le tiroir à l'encontre des moyens de rappel en position d'ouverture de la pince. Cette configuration permet ainsi l'ouverture et l'obturation automatisées de la tubulure pour chaque compartiment du boitier.

Selon une forme de réalisation, le moyen de commande comprend un levier pivotant sur un axe et un électroaimant commandant le déplacement du levier entre ses deux positions actives et inactives. Ainsi, en cas de disfonctionnement, tel qu'une panne de courant, l'électroaimant n'agissant plus sur le levier, celui-ci se place par défaut en position inactive. Les moyens de rappel sollicitent alors le tiroir en position d'obturation de la pince ce qui stoppe l'administration de fluide.

Selon un mode particulier de réalisation, les moyens de verrouillage de chacune des portes comprennent deux pênes pouvant se déplacer pour ouvrir et fermer la porte et un organe de verrouillage conçu pour se déplacer entre une position de déverrouillage de la porte dans laquelle les pênes sont libres de se déplacer pour ouvrir la porte et une position de verrouillage de la porte dans laquelle l'organe de verrouillage vient immobiliser les pênes en position de verrouillage de la porte.

Les moyens de verrouillage comprennent par exemple un dispositif de rappel élastique qui sollicite l'organe de verrouillage en position de verrouillage de la porte.

De façon avantageuse, le moyen de commande du tiroir coopère avec l'organe de verrouillage de sorte que lorsque le moyen de commande se déplace de sa position active à sa position inactive, il entraine le déplacement de l'organe de verrouillage de sa position de verrouillage à sa position de déverrouillage, à l'encontre du dispositif de rappel élastique. Ainsi le moyen de commande initie astucieusement le déplacement de la pince en position d'ouverture de la tubulure dans le même temps qu'il entraine le verrouillage de la porte du compartiment, condamnant l'accès à la pince et sécurisant l'administration du fluide.

De préférence, chaque porte du boitier comprend un organe de déverrouillage comprenant un bouton poussoir disposé sur la porte, l'organe de déverrouillage étant arrangé de sorte que lorsqu'une pression est exercée sur le bouton poussoir, l'organe de déverrouillage entraine le déplacement de l'organe de verrouillage de sa position de verrouillage à sa position de déverrouillage, à l'encontre du dispositif de rappel élastique. Il est ainsi possible de déverrouiller la porte depuis l'extérieur du compartiment de sorte à permettre son ouverture, en cas d'urgence par exemple.

Selon une possibilité, l'organe de déverrouillage comprend une fourchette pivotant autour d'un axe, une première extrémité de la fourchette formant le bouton poussoir et une deuxième extrémité de la fourchette coopérant avec l'organe de verrouillage de sorte à pouvoir entrainer le déplacement de l'organe de verrouillage de sa position de verrouillage à sa position de déverrouillage. Ainsi, la ligne et le boitier présentent une architecture simple, permettant un accès d'urgence à l'intérieur de chacun des compartiments, indépendamment du positionnement du levier en position active ou inactive.

Selon un aspect de l'invention, chaque compartiment comprend un dispositif de détection de gouttes de sorte à déterminer la fin de l'écoulement des gouttes de fluide dans la tubulure.

Le dispositif de détection de gouttes comprend par exemple une cellule optique et/ou un dispositif à ultra son.

Selon une forme de réalisation, la pince comprend une fenêtre dans laquelle la tubulure est susceptible d'être engagée et lorsque la pince est en position d'obturation, la dimension de la fenêtre est inférieure au diamètre de la tubulure et lorsque la pince est en position d'ouverture, la dimension de la fenêtre est supérieure au diamètre de la tubulure. La pince se présente ainsi sous la forme d'un organe d'obturation de tubulure de conception simple et facile à fabriquer.

Selon un deuxième aspect, l'invention concerne un procédé d'utilisation d'une ligne de perfusion multivoie telle que décrite précédemment et qui comprend les étapes successives suivantes :
a) insertion de la pince d'obturation équipée de la tubulure dans le moyen de réception de chaque compartiment,
b) déplacement du moyen de réception d'un compartiment de la position d'obturation à la position d'ouverture de la tubulure par la commande des moyens de commande du déplacement des moyens de réception,
c) verrouillage de la porte du compartiment par les moyens de verrouillage et interdiction d'accès à la pince en position d'ouverture,
d) détection de fin d'écoulement de gouttes du fluide dans la tubulure par le dispositif de détection de gouttes de sorte à déclencher l'étape e).
e) déplacement du moyen de réception de la position d'ouverture à la position d'obturation de la tubulure par la commande des moyens de commande du déplacement des moyens de réception,
f) déverrouillage de la porte du compartiment et autorisation d'accès à la pince en position d'obturation, et
h) répétition des étapes b) à f) jusqu'à la détection de la fin de l'écoulement de gouttes de fluide de chaque compartiment.

Ce procédé permet ainsi l'administration séquencée de différents fluides de façon automatique, sécurisée, sans requérir l'intervention d'un opérateur. L'étape d) permet notamment d'enchainer l'injection des différents fluides sans perte de temps entre chaque séquence. De plus, le fait de détecter la fin de l'écoulement du fluide assure l'administration de la totalité de la substance médicale au patient.

De façon avantageuse, le procédé comprend entre les étapes f) et h) une étape g) de rinçage de la tubulure principale à laquelle les tubulures sont connectées en aval, consistant au déplacement du moyen de réception de la pince équipée de la tubulure du fluide de rinçage en position d'ouverture de la tubulure pendant la durée nécessaire à la délivrance d'une dose prédéfinie de fluide de rinçage. Ainsi, après chaque injection de substance médicale active, la tubulure principale, par laquelle chaque substance médicale est administrée, est rincée. Ceci permet d'une part d'injecter au patient les traces résiduelles de substances actives restant sur les parois des tubulures et d'autre par d'éviter de mixer les substances à administrer.

L'invention sera mieux comprise à l'aide de la description qui suit, en référence aux dessins schématiques annexés représentant le boitier et ses compartiments en différentes positions de fonctionnement et une ligne de perfusion doté du boitier selon l'invention. Dans la suite de la description, les éléments portant les mêmes références numériques sont identiques.
La figure 1 est une vue schématique illustrant la ligne de perfusion selon un mode de réalisation de l'invention.
La figure 2 est une vue en perspective partielle représentant un compartiment du boitier, porte ouverte, en position d'obturation de la tubulure.
La figure 3 est une vue en perspective partielle représentant un compartiment dont la porte est fermée, non verrouillée, en position d'obturation de la tubulure.
La figure 4 est une vue en perspective partielle illustrant la porte d'un compartiment en position non verrouillée.
La figure 5 est une vue en perspective partielle représentant un compartiment dont la porte est fermée, verrouillée, en position d'ouverture de la tubulure.
La figure 6 est une vue en perspective partielle représentant un compartiment en position d'ouverture de la tubulure.
La figure 7 est une vue en perspective partielle illustrant la porte d'un compartiment en position verrouillée.
Les figures 8 et 9 sont des vues en perspective partielle illustrant la porte d'un compartiment comprenant un organe de déverrouillage selon un mode de réalisation particulier de l'invention.

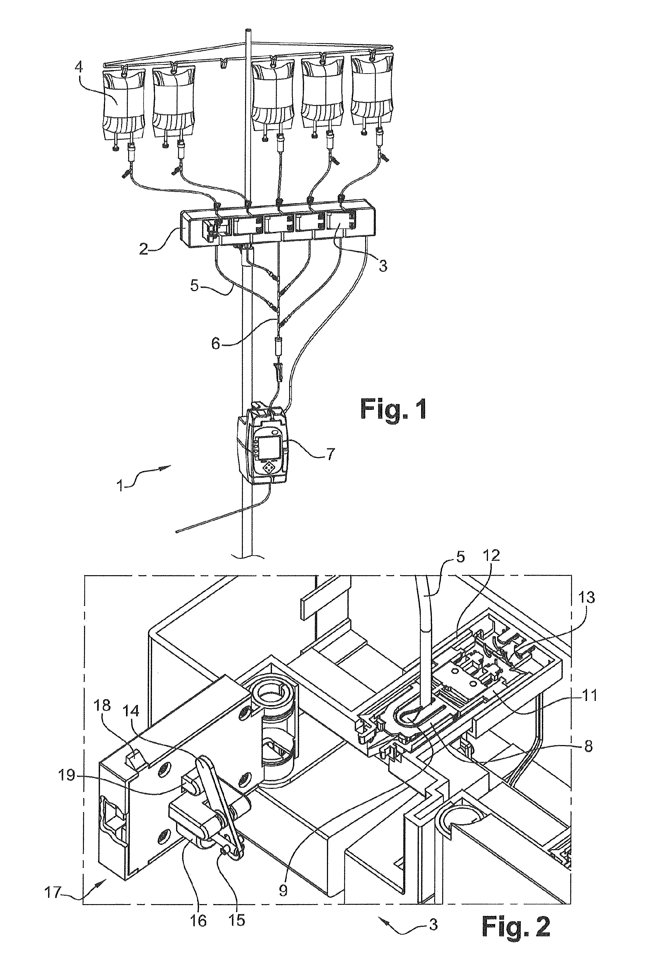

La figure 1 représente une ligne de perfusion multivoie 1 comprenant un boitier 2 doté de plusieurs compartiments 3 et des poches 4 contenant différents fluides à administrer au patient. Chaque poche 4 a été remplie au préalable d'un fluide sous atmosphère stérile pour éviter toute contamination. Le fluide est principalement composé d'un sérum auquel une quantité très précise d'une substance médicale a été ajoutée.

Les poches 4 sont chacune reliées à une tubulure 5 maintenue dans un compartiment 3 permettant l'obturation et l'ouverture automatisées de la tubulure 5 pour la circulation d'un fluide.

Les tubulures 5 sont connectées en aval du boitier 2 à une tubulure principale 6 par laquelle circulent les différents fluides à injecter. La tubulure principale 6 est engagée dans une pompe 7 qui régule le déplacement du fluide dans la ligne de perfusion 1 en coopération avec le boitier 2.

Dans un mode de réalisation non représenté, chaque compartiment 3 comprend un dispositif de détection de gouttes de sorte à déterminer la fin de l'écoulement des gouttes de fluide dans chacune des tubulures 5. Ce dispositif de détection peut être constitué d'un dispositif à ultra son ou d'une cellule optique.

Les figures 2, 3 et 4 représentent le boitier 2 selon un mode de réalisation de l'invention lorsqu'un compartiment 3 est en position d'obturation de la tubulure 5.

La figure 2 illustre un compartiment 3 du boitier 2 en position d'obturation de la tubulure 5, la porte du compartiment 3 ouverte. Le compartiment 3 comprend un moyen de réception dans lequel est insérée une pince 8 qui compresse la tubulure 5 de sorte à interrompre la circulation de fluide dans celle-ci.

La tubulure 5 est typiquement constituée d'un tube souple. Elle est engagée dans une fenêtre 9 ménagée dans la pince 8. Dans la position d'obturation, la tubulure 5 se situe dans la région de la fenêtre 9 qui présente une dimension inférieure au diamètre de la tubulure 5 si bien que celle-ci est pincée et ne permet plus la circulation du fluide.

Comme illustré à la figure 2, le moyen de réception est constitué d'un tiroir 11 mobile qui assure le déplacement de la pince 8 entre une position d'obturation de la tubulure 5 et une position d'ouverture de la tubulure 5.

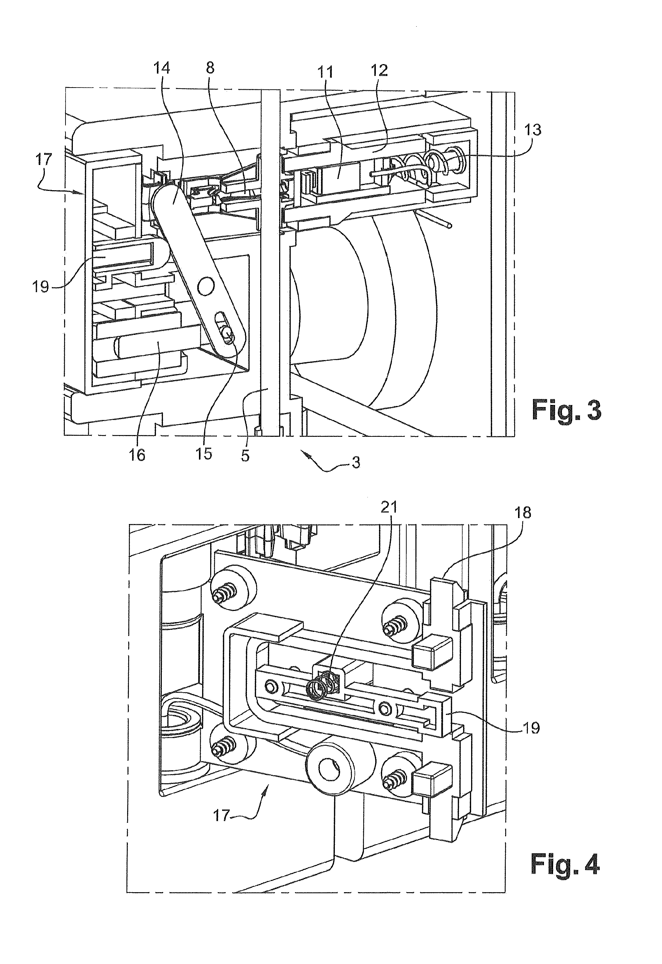

Des portées 12 prévues dans le compartiment 3 permettent de guider le tiroir 11 dans son déplacement et des moyens de rappel élastiques sollicitent le tiroir 11 dans la position d'obturation de la tubulure 5. Dans le mode de réalisation représenté aux figures 2 et 3, les moyens de rappel élastiques sont constitués de ressorts 13 hélicoidaux.

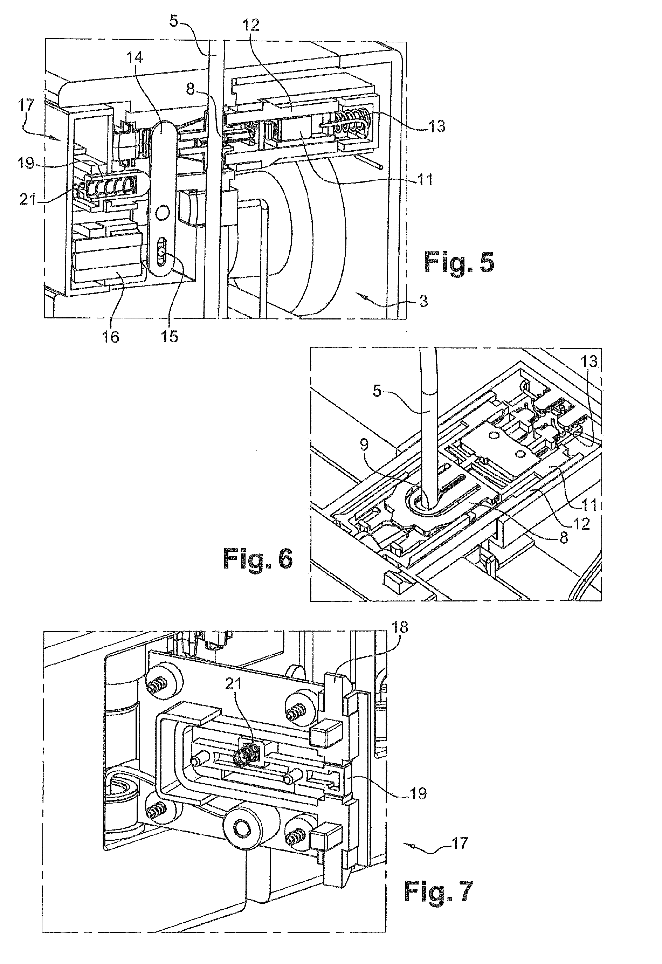

Un moyen de commande du déplacement du tiroir 11 est prévu dans chacun des compartiments 3 du boitier 2. Ce moyen de commande mobile comprend un levier 14 pivotant sur un axe 15 illustré à la figure 2. Le levier 14 peut se déplacer entre une position inactive et une position active sous la commande d'un électroaimant 16. La position inactive est une position inclinée, telle que représenté aux figures 2 et 3, dans laquelle le tiroir 11 est maintenu en position d'obturation de la pince 8 par les ressorts 13. La position active est sensiblement verticale, telle que représenté à la figure 5, dans laquelle le levier 14 pousse le tiroir 11 en position d'ouverture de la pince 8 à l'encontre de la force excercée par les ressorts 13.

Le compartiment 3 comporte une porte 17 représentée ouverte, autorisant l'accès à l'intérieur du boitier 2. Elle est dotée par ailleurs de moyens de verrouillage qui assurent le verrouillage de la porte 17 en position fermée. Ces moyens comprennent deux pênes 18 mobiles qui assurent l'ouverture et la fermeture de la porte 17. Un organe de verrouillage 19 mobile entre une position de verrouillage et de déverrouillage est également prévu. Les moyens de verrouillage comprennent également un dispositif de rappel élastique 21 sollicitant l'organe de verrouillage 19 en position de verrouillage de la porte 17.

Dans le mode de réalisation représenté à la figure 2, le levier 14 est disposé sur la porte 17 de façon à pouvoir coopérer avec l'organe de verrouillage 19. Lorsque le levier 14 est en position inactive, il excerce une contrainte sur l'organe de verrouillage 19 et le maintient en position déverrouillée contre la force exercée par le dispositif de rappel 21.

En se déplaçant à sa position active, le levier 14 diminue la contrainte excercée sur l'organe de verrouillage 19 qui se déplace en position de verrouillage sous l'action du dispositif de rappel 21. Le tiroir 11 est poussé dans le même temps en position d'ouverture de la tubulure 5. La porte 17 du compartiment 3 est donc verrouillée lorsque la tubulure 5 est passante. L'accès à l'intérieur du compartiment 3 est alors impossible, ce qui permet de sécuriser l'administration du fluide.

La figure 3 illustre un compartiment 3 du boitier 2 dont la porte 17 est représentée fermée. Le levier 14 est en position inactive si bien que la pince 8 du tiroir 11 est maintenue en position d'obturation de la tubulure 5 par les moyens de rappel élastiques. L'organe de verrouillage 19 est en position de déverrouillage, la porte 17 du compartiment 3 peut donc être ouverte par un opérateur par le simple actionnement des pênes 18.

La figure 4 représente les moyens de verrouillage de la porte 17 fermée en position déverrouillée. Le levier 14 en position inactive maintient l'organe de verrouillage 19 en position déverrouillée, contre la force exercée par le dispositif de rappel 21. Dans cette position, l'organe de verrouillage 19 autorise le déplacement des pênes 18 l'un vers l'autre et l'ouverture de la porte 17. Le dispositif de rappel élastique 21, tel que représenté dans le mode de réalisation des figures 4 et 7, est constitué d'un ressort.

Les figures 5 , 6 et 7 illustrent le boitier 2 selon un mode de réalisation de l'invention lorsque le compartiment 3 est en position d'ouverture de la tublure.

La figure 5 représente en un compartiment 3 dont la porte 17 est fermée en position verrouillée, la pince 8 en position d'ouverture de la tubulure 5. Le levier 14 est en position active et exerce une pression sur le tiroir 11 de sorte à le maintenir en position d'ouverture de la tubulure 5, à l'encontre de la force des ressorts 13. Le fluide peut ainsi circuler dans la tubulure 5 et être administré au patient.

Dans cette position active, la contrainte excercée par le levier 14 sur l'organe de verrouillage 19 est relâchée. L'organe 19 est alors en position de verrouillage de la porte 17 sous la sollicitation du dispositif de rappel élastique 21. Il interdit l'accès à l'intérieur du compartiment 3 en position d'administration du fluide.

La figure 6 représente le tiroir 11 d'un compartiment 3 retenant la pince 8 en position d'ouverture de la tubulure 5 à l'encontre de la force exercée par les ressorts 13. Dans cette disposition, la tubulure 5 engagée dans la pince 8 se situe dans la région de la fenêtre 9 qui présente une dimension supérieure au diamètre de la tubulure 5. Celle-ci n'est donc pas comprimée et peut laisser circuler le fluide.

La figure 7 représente la porte 17 fermée en position verrouillée. L'organe de verrouillage 19, libéré de la contrainte exercée par le levier 14, est maintenu en position de verrouillage sous la sollicitation du dispositif de rappel 21. Dans cette position, une partie de l'organe de verrouillage 19 est encastré entre les deux pênes 18 les rendant immobiles et de ce fait bloquant l'ouverture de la porte 17. L'accès à l'intérieur du compartiment 3 est donc impossible, ce qui sécurise l'administration du fluide.

Dans l'exemple représenté à la figure 1, il est prévu que ce soit la pompe 7 qui embarque une électronique de contrôle programmable de la position de chacune des pinces 8 d'obturation. Par électronique de contrôle on entend par exemple d'un microprocesseur doté d'une mémoire ROM et/ou RAM, une interface d'entée et de sortie. Pour cela, il peut être est prévu une liaison filaire ou non filaire Bluetooth ou Wifi entre la pompe 7 et le boitier 2.

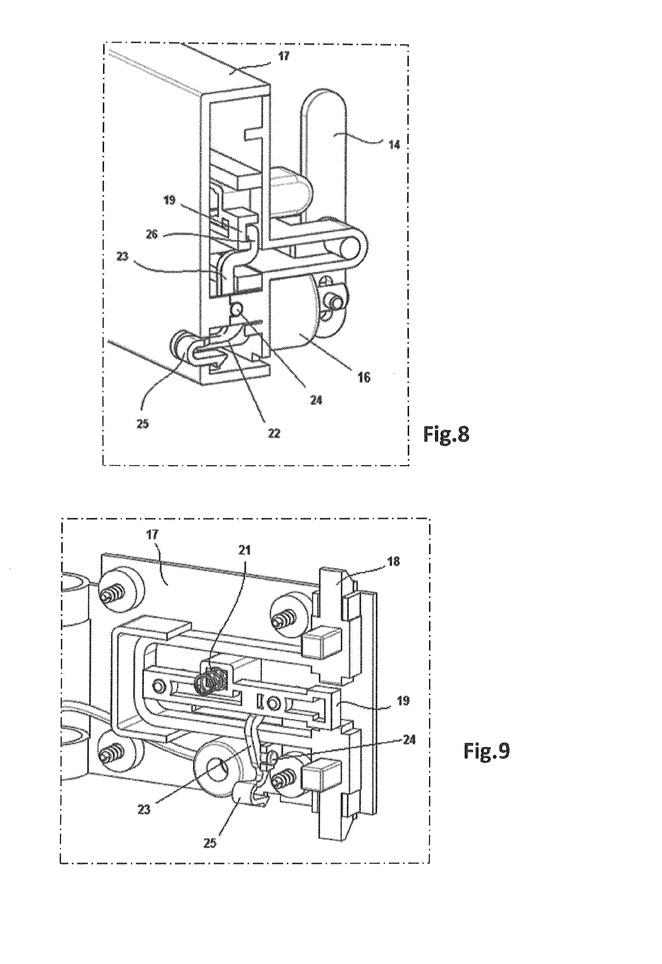

Les figures 8 et 9 illustrent un mode de réalisation particulier d'une porte 17 d'un compartiment 3 comprenant un organe de déverrouillage 22 permettant de déverrouiller la porte 17, quelle que soit la position d'obturation ou d'ouverture de la tubulure 5 dans laquelle se présente la pince 8 du compartiment 3. Cet organe de déverrouillage 22 comprend une fourchette 23 pivotante autour d'un axe 24 et dont une première extrémité comporte un bouton poussoir 25 faisant saillie sur la face extérieure de la porte 17 (figure 8). Une deuxième extrémité 26 de la fourchette 23 opposée au bouton poussoir 25 vient au contact de l'organe de verrouillage 19 de sorte à pouvoir y exercer une pression, à l'encontre du dispositif de rappel 21 élastique. Une pression exercée sur le bouton poussoir 25, par exemple par un opérateur, entraine le pivotement de la fourchette 23 autour de l'axe 24 de sorte que la deuxième extrémité 26 exerce une pression sur l'organe de verrouillage 19. Cette pression engendre le déplacement de l'organe de verrouillage 19 de sa position de verrouillage à sa position de déverrouillage, libérant l'actionnement des pênes 18 (figure 9). Dans ce cas, le déplacement de l'organe de verrouillage 19 est indépendant de la position active ou inactive dans laquelle se présente le levier 14. Ainsi, un opérateur peut déverrouiller manuellement la porte 17 et l'ouvrir, par exemple en situation d'urgence, pour accéder à l'intérieur du compartiment 3 que la pince 8 soit en position d'obturation ou d'ouverture de la tubulure 5. Bien entendu, chacune des portes 17 du boitier 2 peut être munie de l'organe de déverrouillage 22.

Lorsque l'on souhaite utiliser la ligne de perfusion multivoie 1 pour administrer de façon séquencée et automatique différentes substances médicales à un patient, un opérateur ouvre tout d'abord les portes 17 de chacun des compartiments 3 pour donner un libre l'accès au tiroir 11. Les pinces 8, pourvues chacune d'une tubulure 5 reliée à une poche 4, sont insérées dans le tiroir 11 de chaque compartiment 3. Les ressorts 13 maintiennent les tiroirs 11 en position d'obturation de la pince 8 et interdisent la circulation du fluide. Les portes 17 des compartiments 3 sont ensuite refermées en actionnant les pênes 18.

L'opérateur entre alors les paramètres de perfusion par la pompe 7 c'est-à-dire ordre de perfusion des poches, débit de chaque poche, etc.

Le moyen de commande du déplacement du tiroir 11 d'un compartiment 3 associé à un fluide à délivrer actionne l'électroaimant 16 du compartiment 3 correspondant. Celui-ci actionne le déplacement du levier 14 de sa position inactive à sa position active. Le levier 14 excerce alors une pression sur le tiroir 11 qui se déplace contre la force des ressorts 13 en position d'ouverture de la pince 8. La tubulure 5 maintenue dans le compartiment 3 reste immobile tandis que la pince 8 se déplace de sorte à ce que la fenêtre 9 de la pince 8 passe d'une position de compression de la tubulure 5 à une position où elle libère la tubulure 5. Celle-ci laisse alors circuler le fluide contenu dans la poche 4 pour une administration au patient.

Dans le même temps, le déplacement du levier 14 en position active relache la contrainte sur l'organe de verrouillage 19 de la porte 17. L'organe de verrouillage 19, sollicité par le dispositif de rappel 21, vient s'intercaler entre les pênes 18 de sorte à les immobiliser, verrouillant ainsi la porte 17 et l'accès à la tubulure 5 en phase passante.

Une fois la poche 4 et la tubulure 5 vidées, le dispositif de détection du compartiment 3 détecte la fin de l'écoulement de gouttes indiquant que la substance médicale contenue dans la poche 4 a été totalement injectée au patient. Les moyens de commande du déplacement du tiroir 11 actionnent le levier 14 qui revient en position inactive. Le tiroir 11 revient alors en position d'obturation de la tubulure 5 et l'organe de verrouillage 19 se déplace pour retrouver sa position déverrouillée. La porte 17 peut ainsi être ouverte par un opérateur.

Dans le même temps, les moyens de commande du déplacement du tiroir 11 du compartiment 3 du second fluide à injecter actionne l'électroaimant 16 du compartiment 3 correspondant. Le levier 14 associé se déplace en position active entrainant le verrouillage de la porte 17 et le déplacement de la pince 8 en position d'ouverture de la tubulure 5. Puis, lorsque la fin d'écoulement de gouttes de fluide est détectée, l'électroaimant 16 actionne le levier 14 qui pivote et revient à sa position inactive. Le tiroir 11 revient en position d'obturation de la tubulure 5 et la porte 17 est à nouveau déverrouillée.

Cette séquence d'étapes est reproduite de façon automatique, en conformité avec les instructions introduites dans l'électronique de contrôle intégrée à la pompe 7, pour chaque fluide médical à injecter.

Lorsqu'il est impératif que les fluides ne soient pas mélangés, il est prévu une étape de rinçage de cette tubulure principale 6 par laquelles tous les fluides circulent avant d'être administrés au patient. A cet effet, après la détection de fin d'écoulement de gouttes et avant l'ouverture d'une nouvelle tubulure 5 permettant l'adminstration d'un nouveau fluide médical au patient, un électroaimant 16 d'un compartiment 3 associé à un fluide de rinçage actionne l'ouverture de la tubulure 5 correspondante pour délivrer une dose prédéfinie de fluide de rinçage. Cette dose prédéfinie de fluide peut être calculée à partir de la section et de la longueur de la tubulure principale 6 à rincer. Le temps de rinçage peut être calculé selon le débit utilisé.

Ainsi, la ligne de perfusion multivoie 1 selon l'invention est peu coûteuse et permet l'adminstration séquentielle sécurisée et automatique de différents fluides à un patient. Comme il va de soi, l'invention ne se limite pas aux formes d'exécution et d'utilisation de la ligne de perfusion 1 décrites ci-dessus à titre d'exemple, elle en embrasse au contraire toutes les variantes.

## Revendications

1. Ligne de perfusion multivoie (1) **caractérisée en ce qu'**elle comprend
- un boitier (2) comprenant plusieurs compartiments (3) susceptibles de maintenir chacun une tubulure (5) pour fluide engagée dans une pince (8) d'obturation, chacun des compartiments (3) comportant un moyen de réception de la pince (8) conçu pour se déplacer entre:
une position d'obturation de la tubulure (5) dans laquelle la pince (8) insérée dans le moyen de réception compresse la tubulure (5) de sorte à interrompre la circulation du fluide dans la tubulure (5), et
une position d'ouverture de la tubulure (5) dans laquelle la pince (8) libère la tubulure (5) de sorte à autoriser la circulation du fluide dans la tubulure (5),
le boitier (2) comprenant des moyens de commande du déplacement de chaque moyen de réception,
- plusieurs poches (4) destinées à contenir un fluide, chacune des poches (4) étant reliée à un compartiment (3) du boitier (2) par l'intermédiaire d'une tubulure (5), chacune des tubulures (5) étant reliées en aval du boitier (2) à une tubulure principale (6) engagée dans la pompe (7), la pompe (7) régulant le débit du fluide dans la ligne (1) de perfusion, et
- des moyens de contrôle programmables de la position de chacune des pinces (8) d'obturation permettant le séquençage automatique de la perfusion du fluide des différentes poches (4) de la ligne de perfusion multivoie (1).

2. Ligne de perfusion multivoie (1) selon la revendication 1, **caractérisée en ce que** les moyens de contrôle programmables sont intégrés à la pompe (7).

3. Ligne de perfusion multivoie (1) selon l'une des revendications 1 à 2, **caractérisée en ce que** chacun des compartiments (3) comprend une porte (17) équipée de moyens de verrouillage aptes à verrouiller la porte (17) pour interdire l'accès à la pince (8) d'obturation en position d'ouverture de la tubulure (5).

4. Ligne de perfusion multivoie (1) selon l'une des revendications 1 à 3, **caractérisée en ce que** le moyen de réception de la pince (8) d'obturation comprend un tiroir (11) mobile dans lequel la pince (8) est insérée et **en ce que** chaque compartiment (3) comprend des portées (12) sur lesquelles le tiroir (11) mobile peut se déplacer entre la position d'ouverture et la position d'obturation de la pince (8).

5. Ligne de perfusion multivoie (1) selon la revendication 4, **caractérisée en ce que** chaque compartiment (3) comprend des moyens de rappel élastiques qui sollicitent le tiroir (11) vers la position d'obturation de la pince (8).

6. Ligne de perfusion multivoie (1) selon l'une des revendications 4 à 5, **caractérisée en ce que** chaque compartiment (3) comprend un moyen de commande du déplacement du tiroir (11), mobile entre une position inactive dans laquelle le tiroir (11) est maintenu en position d'obturation de la pince (8) par des moyens de rappel élastiques et une seconde position active dans laquelle le moyen de commande pousse le tiroir (11) à l'encontre des moyens de rappel en position d'ouverture de la pince (8).

7. Ligne de perfusion multivoie (1) selon la revendication 6, **caractérisée en ce que** le moyen de commande comprend un levier (14) pivotant sur un axe (15) et un électroaimant (16) commandant le déplacement du levier (14) entre ses deux positions actives et inactives.

8. Ligne de perfusion multivoie (1) selon l'une des revendications 3 à 7, **caractérisée en ce que** les moyens de verrouillage de chacune des portes (17) comprennent deux pênes (18) pouvant se déplacer pour ouvrir et fermer la porte (17) et un organe de verrouillage (19) conçu pour se déplacer entre une position de déverrouillage de la porte (17) dans laquelle les pênes (18) sont libres de se déplacer pour ouvrir la porte (17) et une position de verrouillage de la porte (17) dans laquelle l'organe de verrouillage (19) vient immobiliser les pênes (18) en position de verrouillage de la porte (17).

9. Ligne de perfusion multivoie (1) selon la revendication 8, **caractérisée en ce que** les moyens de verrouillage comprennent un dispositif de rappel (21) élastique qui sollicite l'organe de verrouillage (19) en position de verrouillage de la porte (17).

10. Ligne de perfusion multivoie (1) selon la revendication 9, **caractérisée en ce que** le moyen de commande du tiroir (11) coopère avec l'organe de verrouillage (19) de sorte que lorsque le moyen de commande se déplace de sa position active à sa position inactive, il entraine le déplacement de l'organe de verrouillage (19) de sa position de verrouillage à sa position de déverrouillage, à l'encontre du dispositif de rappel élastique (21).

11. Ligne de perfusion multivoie (1) selon l'une des revendications 9 à 10 **caractérisée en ce que** chaque porte (17) du boitier (2) comprend un organe de déverrouillage (22) comprenant un bouton poussoir (25) disposé sur la porte (17), l'organe de déverrouillage (22) étant arrangé de sorte que lorsqu'une pression est exercée sur le bouton poussoir (25), l'organe de déverrouillage (22) entraine le déplacement de l'organe de verrouillage (19) de sa position de verrouillage à sa position de déverrouillage, à l'encontre du dispositif de rappel élastique (21).

12. Ligne de perfusion multivoie (1) selon la revendication 11, **caractérisée en ce que** l'organe de déverrouillage (22) comprend une fourchette (23) pivotant autour d'un axe (24), une première extrémité de la fourchette (23) formant le bouton poussoir (25) et une deuxième extrémité de la fourchette (26) coopérant avec l'organe de verrouillage (19) de sorte à pouvoir entrainer le déplacement de l'organe de verrouillage (19) de sa position de verrouillage à sa position de déverrouillage.

13. Ligne de perfusion multivoie (1) selon l'une des revendications 1 à 12, **caractérisée en ce que** chaque compartiment (3) comprend un dispositif de détection de gouttes de sorte à déterminer la fin de l'écoulement des gouttes de fluide dans la tubulure (5).

14. Procédé d'utilisation d'une ligne de perfusion multivoie (1) selon la revendication 13 **caractérisé en ce qu'**il comprend les étapes successives suivantes :
a) insertion de la pince (8) d'obturation équipée de la tubulure (5) dans le moyen de réception de chaque compartiment (3),
b) déplacement du moyen de réception d'un compartiment (3) de la position d'obturation à la position d'ouverture de la tubulure (5) par la commande des moyens de commande du déplacement des moyens de réception
c) verrouillage de la porte (17) du compartiment (3) par les moyens de verrouillage et interdiction d'accès à la pince (8) en position d'ouverture,
d) détection de fin d'écoulement de gouttes du fluide dans la tubulure (5) par le dispositif de détection de gouttes de sorte à déclencher l'étape e).
e) déplacement du moyen de réception de la position d'ouverture à la position d'obturation de la tubulure (5) par la commande des moyens de commande du déplacement des moyens de réception
f) déverrouillage de la porte (17) du compartiment (3) et autorisation d'accès à la pince (8) en position d'obturation
h) répétition des étapes b) à f) jusqu'à la détection de la fin d'écoulement de gouttes de fluide de chaque compartiment.

15. Procédé d'utilisation d'une ligne de perfusion multivoie (1) selon la revendication 14 **caractérisé en ce qu'**il comprend entre les étapes f) et h) une étape g) de rinçage de la tubulure principale (6) à laquelle les tubulures (5) sont connectées en aval, consistant au déplacement du moyen de réception de la pince (8) équipée de la tubulure (5) du fluide de rinçage en position d'ouverture de la tubulure (5) pendant la durée nécessaire à la délivrance d'une dose prédéfinie de fluide de rinçage.
